# FASCICULE DE BREVET EUROPEEN

(11) **EP 2 315 578 B1**
(45) Date de publication et mention de la délivrance du brevet: **23.08.2017**
(21) Numéro de dépôt: 09784278.5
(22) Date de dépôt: 20.07.2009
(51) Int. Cl.: A61K 8/97, A61Q 19/08

(54) **COMPOSITION COSMETIQUE COMPRENANT UN EXTRAIT AQUEUX DE CHRYSOPHYLLUM CAINITO**
KOSMETISCHE ZUSAMMENSETZUNG MIT EINEM WÄSSRIGEN EXTRAKT VON CHRYSOPHYLLUM CAINITO
COSMETIC COMPOSITION COMPRISING AN AQUEOUS EXTRACT OF CHRYSOPHYLLUM CAINITO

(30) Priorité: 22.07.2008 FR 0804170
(43) Date de publication de la demande: 04.05.2011
(73) Titulaire: Laboratoires Clarins, 95304 Cergy Pontoise Cedex (FR)
(72) Inventeur: COURTIN, Olivier, F-92200 Neuilly Sur Seine (FR)
(74) Mandataire: Gevers & Orès
(86) Numéro de dépôt international: PCT/FR2009/000890
(87) Numéro de publication internationale: WO 2010/010248

(56) Documents cités:
- WO-A-2006/009418
- DATABASE WPI Week 200140 Thomson Scientific, London, GB; AN 2001-379481 XP002518051, & JP 2001 122732 A (ICHIMARU PHARCOS INC) 8 mai 2001 (2001-05-08) -& DATABASE CA [Online] CHEMICAL ABSTRACTS SERVICE, COLUMBUS, OHIO, US; 8 mai 2001 (2001-05-08), OHARA, MITSUHARU ET AL: "Cosmetics, bath preparations, and detergents containing moisturizing extracts of Sapotaceae plants", XP002518050, extrait de STN Database accession no. 2001:326220
- L.Einbond et al.: "Anthocyanin antioxidants from edible fruits", Food Chemistry, vol. 84 2004, pages 23-28, XP002518048, Extrait de l'Internet: URL:http://home.earthlink.net/~phytochemis try/archive/einbond.pdf [extrait le 2009-03-05]
- XIAO-DONG LUO ET AL.: "Polyphenolic antioxidants from the fruits of chrysophyllum cainito (star apple)", Journal of Agricultural and Food Chemistry, vol. 50, no. 2 2002, pages 1379-1382, XP002518049, Extrait de l'Internet: URL:http://pubs.acs.org/doi/pdf/10.1021/jf 011178n [extrait le 2009-03-06]
- CHOI ET AL: "Cosmeceuticals", SEMINARS IN CUTANEOUS MEDICINE AND SURGERY, W.B. SAUNDERS, PHILADELPHIA, US, vol. 25, no. 3, 1 septembre 2006 (2006-09-01), pages 163-168, XP005703032, ISSN: 1085-5629
- "APPLICATIONS OF OILS EXTRACTED FROM AFRICAN STAR APPLE (CHRYSOPHYLLUM AFRICANUM), HORSE EYE BEAN (MUCUNA SLOANEI) AND AFRICAN PEAR (DACRYODES EDULIS) SEEDS", BIORESOURCE TECHNOLOGY, ELSEVIER, GB, vol. 59, no. 2/03, 1 janvier 1997 (1997-01-01), pages 259-261, XP001097250, ISSN: 0960-8524
- DATABASE BIOSIS [Online] BIOSCIENCES INFORMATION SERVICE, PHILADELPHIA, PA, US; 2002, BAUTISTA-BANOS SILVIA ET AL: "Antifungal activity of leaf and stem extracts from various plant species on the incidence of Colletotrichum gloeosporioides of papaya and mango fruit after storage", XP002518203, Database accession no. PREV200200506847 & REVISTA MEXICANA DE FITOPATOLOGIA, vol. 20, no. 1, 2002, pages 8-12, ISSN: 0185-3309
- Pino J. et al.: "Volatile constituents of star apple (Chrysophyllum cainito L.) from Cuba", FLAVOUR AND FRAGRANCE JOURNAL, vol. 17, no. 5 2002, XP002518346, Extrait de l'Internet: URL:http://www3.interscience.wiley.com/cgi -bin/fulltext/93514196/PDFSTART [extrait le 2009-03-09]
- BOULANGER ET AL: "Vieillissement: role et controle de la glycation", REVUE DE MEDECINE INTERNE, CMR, ASNIERES, FR, vol. 28, no. 12, 21 November 2007 (2007-11-21), pages 832-840, XP022355267, ISSN: 0248-8663, DOI: 10.1016/J.REVMED.2007.05.019
- WOLFF S P ET AL: "Protein glycation and oxidative stress in diabetes mellitus and ageing", FREE RADICAL BIOLOGY AND MEDICINE,, vol. 10, no. 5, 1 January 1991 (1991-01-01), pages 339-352, XP023548530, ISSN: 0891-5849, DOI: 10.1016/0891-5849(91)90040-A [retrieved on 1991-01-01]

## Description

La présente invention concerne l'utilisation d'une composition cosmétique comprenant au moins 0,1% d'extrait aqueux de la graine, de la pulpe et du péricarpe de fruit de Chrysophyllum cainito en poids de matière sèche par rapport au poids total de la composition pour prévenir et/ou retarder et/ou lutter contre le relâchement cutané et la perte d'élasticité des fibres cutanées, par exemples l'enveloppe cutanée des seins. En particulier, cette composition protège la fonctionnalité des cellules cutanées en inhibant la glycation des protéines, en particulier de la vimentine dans les fibroblastes dermiques. La glycation est un processus non enzymatique faisant intervenir un ose (glucose ou ribose) qui réagit selon la réaction de Maillard avec un groupement aminé d'un résidu d'acide aminé (comme par exemple la lysine), particulièrement un résidu d'acide aminé d'une protéine, pour former une base de Schiff. Celle-ci, après un réarrangement moléculaire dit d'Amadori, peut conduire, par une succession de réactions, à un pontage, particulièrement intramoléculaire comme par exemple de type pentosidine.

Ce phénomène se caractérise par l'apparition de produits de glycation dont la teneur augmente de façon régulière en fonction de l'âge. Les produits de glycation sont par exemple la pyrraline, la carboxyméthyl-lysine (CML), la pentosidine, la crossline, la N^{ε}-(carboxyéthyl) -lysine (CEL), le dimère de glyoxal-lysine, le dimère de méthylglyoxal-lysine, la 3DG-ARG imidazolone, les versperlysines A, B, C, la thréosidine ou encore les produits finaux de glycosylation avancée (ou AGEs pour Advanced Glycation End Products)).

La glycation des protéines est donc un phénomène universel, bien connu au niveau de la peau, particulièrement au niveau de sa composante dermique, et principalement au niveau des fibres de collagène. La glycation du collagène augmente en effet de façon régulière avec l'âge, entraînant une augmentation régulière de la teneur de la peau en produits de glycation.

Les AGEs constituent un groupe hétérogène de structures, tandis que les adduits carboxyméthyliques de la lysine (CML) sont les plus répandus *in vivo.* Les CML avec d'autres AGEs s'accumulent pendant le processus de vieillissement intrinsèque menant au raidissement et à une perte d'élasticité dans les tissus comme la peau et la paroi des vaisseaux.

On a longtemps pensé que les AGEs résultent principalement de la réaction entre les protéines et le glucose extracellulaires. Cependant, les études récentes indiquent que la formation intracellulaire d'AGEs à partir des composés dicarbonyl résultant de l'auto-oxydation du glucose est beaucoup plus importante comparée à la formation extracellulaire (Shinohara, M et al (1998) J. Clin. Investig. 101, 1142-1147). Ces composés dicarbonyl sont le glyoxal, le méthylglyoxal, et le déoxyglucosone 3, qui sont des substrats pour les réductases. Le glyoxal et le méthylglyoxal sont détoxifiés par le système de glyoxalase. Les AGEs intracellulaires induisent un stress oxydant, activent NF-κB et le hème oxygénase, produisent des produits de peroxydation lipidique.

Une étude récente (Thomas Kueper et al. J. Biol. Chem., Vol. 282, Issue 32, 23427-23436, August 10, 2007) a identifié la vimentine, protéine de filament intermédiaire, comme cible principale de formation des CML (carboxyméthyl-lysine), dans les fibroblastes cutanés humains. Les filaments intermédiaires sont l'un des éléments structuraux majeurs du cytosquelette en plus des microfilaments d'actine et des microtubules. Toutes les protéines de filament intermédiaire ont une structure secondaire commune, se composant d'un domaine hélicoïdal central d'environ 310 acides aminés qui est flanqué par des domaines non alpha hélicoïdaux de taille variable. La vimentine est requise pour de nombreuses fonctions cellulaires essentielles comme la motilité des cellules, la migration chimiotactique, et la cicatrisation. La modification de la vimentine a comme conséquence une redistribution de la vimentine en agrégat périnucléaire. Il est évident que l'impact biologique de la modification de la vimentine est lié à la perte de capacité contractile des fibroblastes provoquée par la rupture structurale du système de filament intermédiaire accélérant finalement le processus du vieillissement.

Ainsi la glycation non enzymatique des protéines représente la première cause de modification du collagène cutané. L'une des conséquences importantes de la glycation des protéines est la création de radicaux libres. En effet, lorsqu'elles sont touchées par la glycation, les protéines réagissent avec l'oxygène et provoquent la formation de radicaux de type superoxyde. Ces derniers sont capables d'initier la dégradation de protéines, d'altérer les structures membranaires, désorganisant au final la matrice extra-cellulaire ainsi que tous ses composants.

Ainsi, une composition cosmétique contenant des actifs capables de freiner la glycation des protéines permet de lutter contre le vieillissement cutané. L'apparition des rides, la diminution de la tonicité cutanée étant le reflet du vieillissement du derme et notamment de ses propriétés mécaniques.

En particulier l'enveloppe cutanée des seins est, chez la femme particulièrement sensible au vieillissement. En effet, Le sein est un organe pair reposant sur les muscles pectoraux. La glande mammaire est constituée de lobules graisseux et d'une vingtaine de lobules glandulaires placés au plus profond de la glande. Les lobules graisseux donnent au sein sa consistance souple et sa forme. Les lobules glandulaires sont chargés de la sécrétion du lait acheminé par les canaux galactophores vers le sinus galactophore débouchant sur le mamelon. Les canaux excréteurs sont entourés et maintenus par des travées de tissu conjonctif. Le mamelon et l'aréole sont un point de fixation du sein mais la peau est le moyen principal de suspension de la glande. La peau est reliée à la face superficielle de la glande par les ligaments de Cooper. La musculation des pectoraux ne permet pas de modifier la forme ni de remodeler les seins.

Avec l'âge, les influences hormonales, les variations de poids mais aussi les expositions solaires, l'absence de soutien-gorge etc... les fibres élastiques de la peau peuvent se rompre, les ligaments de Cooper se distendre. La peau perdant sa tonicité, les seins s'affaissent : ce phénomène est appelé ptôse. La ptôse mammaire traduit un glissement avec affaissement de la glande et une distension exagérée de l'enveloppe cutanée. Il faut donc agir sur cette enveloppe cutanée en protégeant les protéines de la glycation pour prévenir le vieillissement des seins.

La demanderesse a mis en évidence de manière surprenante qu'un extrait de Chrysophyllum cainito inhibe la glycation des protéines. Le Chrysophyllum cainito est un arbre de la famille des Sapotaceae originaire des grandes Antilles. Ses fruits ronds coupés en deux laissent apparaître une pulpe gélatineuse blanchâtre ou pourpre enserrant dans une loge étoilée à 8 ou 9 branches, 4 à 10 graines aplaties. La pulpe sucrée est plus ou moins liquide selon les variétés.

On connaît le document WO 2006/009418 qui décrit des extraits de graines de sapotacées hydrolysés enzymatiquement pour donner des glycosides cyanogènes utilisables en cosmétique. On connaît également le document WO 2006/009417 qui décrit un procédé d'obtention de lipides à partir de graines de sapotacées. Le document JP 2001 122732 décrit des compositions cosmétiques contenant des extraits de plantes de la famille des sapotacées, ces compositions ayant une activité hydratante capable de prévenir les désagréments liés aux peaux sèches telles que inflammations, démangeaisons. Le document L.Einbond et al., Food Chemistry, 84, 2004, 23-28 décrit l'identification de la présence d'antioxydants, en particulier d'anthocyanes, dans le fruit de Chrysophyllum cainito. Le document Xiao-Dong Luo et al., J. Agric. Food Chem. 2002, 50, 1379-1382, décrit l'identification de la présence d'antioxydants, en particulier de polyphénols, dans le fruit de Chrysophyllum cainito. Le document Choi et al., « Cosmeceuticals » Seminars in Cutaneous Medicine and Surgery, W.B.Saunders, Philadelphia, 25(3), 2006, 163-168, décrit l'intérêt des antioxydants - et plus particulièrement des polyphénols - dans des produits cosmétiques anti-âge. L'extrait de Chrysophyllum cainito de la présente invention se distingue des extraits de l'art antérieur par le fait qu'il s'agit d'un extrait aqueux du fruit total c'est-à-dire de la graine, de la pulpe et du péricarpe du fruit dont la composition chimique est différente des extraits de l'art antérieur.

La Demande JP 2001-122732 A décrit également des compositions cosmétiques contenant des extraits de plantes appartenant à la famille des Sapotaceae tel que le Chrysophylum cainito, pour prévenir les affections des peaux sèches.

La Demande WO 2006/009418 décrit aussi une méthode d'obtention d'extrait de génines et de sapogénines par hydrolyse enzymatique de graines issues de la famille des Sapotaceae tel que le Chrysophylum cainito.
Et aucun document ne divulgue ou ne suggère un effet d'un extrait de Chrysophyllum cainito sur la glycation des protéines, et en particulier sur la ptôse des seins.

La composition cosmétique, objet de la présente invention contient un extrait aqueux de fruit de Chrysophyllum cainito. De façon avantageuse, cet extrait comprend un extrait de péricarpe et de la pulpe du fruit de Chrysophyllum cainito. De préférence il s'agit d'un extrait sec obtenu selon un procédé comprenant au moins les étapes suivantes :
- broyage des fruits secs en coupe
- ajout d'eau (température ambiante) afin de couvrir les fruits
- macération pendant au moins 24 h
- filtration primaire

Cet extrait sec est avantageusement obtenu selon le protocole suivait :
(i) Extraction aqueuse des fruits de Chrysophyllum cainito :
   - broyage des fruits secs en coupe
   - ajout d'eau (température ambiante) afin de couvrir les fruits
   - agitation lente
   - macération pendant au moins 24 h
   - filtration primaire
(ii) Concentration :
   - mesure de matière sèche contenue
   - passage au concentrateur (basse température, basse pression)
(iii) Ajout de maltodextrine et stérilisation
(iiii) Séchage et débactérisation .
   - atomisation du concentrat sur la tour d'atomisation
   - tamisage de la poudre obtenue et conditionnement
   - traitement par ionisation pour débactériser.

L'extrait sec de Chrysophyllum cainito utilisé dans la composition selon l'invention, est une poudre beige d'odeur caractéristique. Il présente les caractéristiques analytiques suivantes :
- solubilité = à 10 % dans l'eau.
- humidité ≤ 10%.
- pH = 5-7.
- granulométrie ≤500 microns.
- sucres totaux = 20-50 %/ matière sèche.
- polyphénols (exprimé en catéchine) = 0.1-1 % / matière sèche.

De préférence la composition cosmétique selon l'invention comprend de 0,1 à 10 % d'extrait de Chrysophyllum cainito en poids de matière sèche par rapport au poids total de la composition. Avantageusement, la composition comprend de 0,1 à 5 % en poids d'extrait de Chrysophyllum cainito en poids de matière sèche par rapport au poids total de la composition.

Les compositions selon l'invention peuvent encore comprendre un ou plusieurs agents de formulation ou additifs d'usage connu et classique dans les compositions cosmétiques et dermatologiques tels que, à titre d'exemples et de façon non limitative, des adoucissants, des colorants, des actifs filmogènes, des tensioactifs, des parfums, des conservateurs, des émulsionnants, des huiles, des glycols, des vitamines comme la vitamine E, des filtres UV, etc... Grâce à ses connaissances en matière de cosmétique, l'homme du métier saura quels agents de formulation ajouter aux compositions de l'invention et en quelles quantités en fonction des propriétés recherchées.

Les compositions selon l'invention peuvent se présenter sous toute forme connue de l'homme du métier dans le domaine de la cosmétologie et de la dermatologie sans autre restriction galénique que l'application sur le visage et sur le corps. De façon avantageuse, les compositions selon l'invention se présentent sous la forme d'un gel, d'une crème, d'une lotion, d'une huile, d'un lait, d'un spray, etc...

La composition cosmétique selon l'invention peut être utilisée pour prévenir, pour retarder ou pour lutter contre le vieillissement de la peau et/ou l'apparition des signes de vieillissement de la peau. On entend par signes de vieillissement de la peau : les rides, les ridules, le relâchement cutané, la perte d'élasticité des fibres cutanées, une peau flétrie, une peau amincie, et une peau terne et/ou sans éclat.

La composition cosmétique selon l'invention peut être utilisée comme agent anti glycation des protéines.

La composition cosmétique selon l'invention peut être utilisée pour prévenir et/ou retarder et/ou corriger la ptose des seins.

L'invention a encore pour objet un procédé de traitement cosmétique comprenant l'application d'une composition telle que décrite ci-dessus, qui comporte au moins un extrait aqueux de Chrysophyllum cainito et un support cosmétique, sur la peau. Ce procédé est destiné à prévenir, retarder ou lutter contre le vieillissement de la peau et/ou l'apparition des signes de vieillissement de la peau, à prévenir et/ou retarder et/ou corriger la ptose des seins, à prévenir ou traiter la glycation des protéines de la peau.

Il est recommandé de faire l'application une ou plusieurs fois par jour (de une à cinq fois par jour en règle générale), sur une durée de quelques jours à quelques mois. L'application peut être localisée sur les zones du corps qui subissent plus particulièrement les effets du vieillissement, telles que le visage et les seins, l'abdomen, mais elle peut être faite sur le corps tout entier. Cette application est préférentiellement accompagnée d'un massage de la peau.

Les exemples ci-après concernent, d'une part l'évaluation de l'inhibition de la glycation des protéines par un extrait de Chrysophyllum cainito, et d'autre part des compositions objets de la présente invention.

Les exemples font référence aux figures suivantes dans lesquelles :
- La figure 1 représente la mesure de l'intensité de fluorescence en fonction des actifs présents dans le milieu de culture : glyoxal seul ou glyoxal avec un inhibiteur de glycation.
- La figure 2 représente le pourcentage d'inhibition de la glycation par rapport au témoin non traité en fonction des actifs présents dans le milieu de culture : glyoxal avec un inhibiteur de glycation.
- La figure 3 représente les photographies en microscope à fluorescence des fibroblastes au grossissement 200 fois.
- La figure 4 représente un agrandissement des clichés de la figure 3 au grossissement 400 fois.

### I.EVALUATION DE L'INHIBITION DE LA GLYCATION DES PROTEINES PAR UN EXTRAIT DE CHRYSOPHYLLUM CAINITO

### A. MATERIEL ET METHODE

### 1) Méthode

Il s'agit d'évaluer l'activité inhibitrice de l'extrait de Chrysophyllum cainito vis à vis des CML (adduits carboxyméthyliques de la lysine) induits par le glyoxal ainsi que son effet protecteur de la vimentine, cible de formation des CML, au niveau d'une culture des fibroblastes humains dermiques.

Des fibroblastes humains normaux adultes ont été cultivés à 37 °C et 5%CO₂ sur lamelle en boite de Pétri de diamètre 60 à raison de 30000 cellules par lamelle. Le milieu d'incubation est le milieu DMEM (Dulbecco milieu essentiel modifié) complété avec 10% de sérum de veau foetal et la pénicilline/streptomycine (50 µg/ml). Les fibroblastes ont été incubés pendant 7 jours dans le milieu décrit ci-dessus supplémenté avec 200 µM de glyoxal (dérivé du glucose). La culture ainsi établie constitue un modèle de glycation *in vitro* pertinent.

### 2) Matériel

Les produits à l'essai sont les suivants
- contrôle (sans Glyoxal)
- témoin non traité
- Aminoguanidine 10mM (référence positive)
- Chrysophyllum cainito à 0.1%
- Chrysophyllum cainito à 0.25%
- Chrysophyllum cainito à 0.5%

Pour appréhender un effet inhibiteur de la glycation, l'extrait de Chrysophyllum cainito est ajouté en même temps que le glyoxal. Pour valider l'expérimentation, l'aminoguanidine, inhibiteur connu, est utilisée comme référence positive. Un contrôle milieu seul sans glyoxal est ajouté.

A la fin de l'incubation (J7), chaque tapis cellulaire est ensuite rincé, fixé au méthanol à -20°C avant de procéder à la révélation de la vimentine ou de CML par immunofluorescence.

### 3) Immunomarquage

Les cellules ont été lavées au PBS (tampon salin phosphaté) et perméabilisées avec 0,1% Triton X-100 pendant 10 minutes. Les sites non spécifiques sont saturés par une solution de sérum albumine bovine 2 % (PBS-BSA 2 %) pendant 10 minutes à température ambiante. Les cellules ont été alors incubées avec de l'anticorps primaire dans 1% BSA pour 1h (des anticorps primaires détectant la vimentine (dilution 1:100) ou CML (dilution 1:50)). Après incubation, les cellules ont été lavées au PBS puis incubées pour 1 h avec la solution de 1% BSA contenant l'anticorps secondaire spécifique lié à un fluorochrome FITC (Fluorescein-5-Iso Thio Cyanate) ou TRITC (Tetra methyl Rhodamine Iso Thio Cyanate) et dirigé contre le 1^{er} anticorps. Avant la lecture, 4 rinçages au PBS sont réalisés.

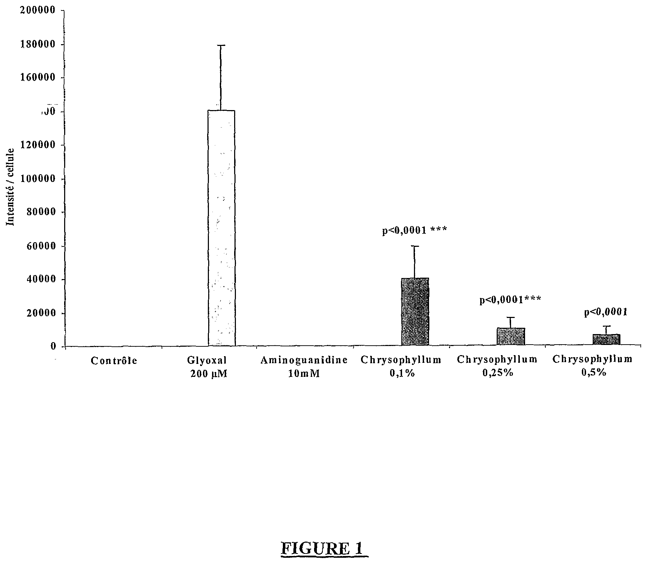

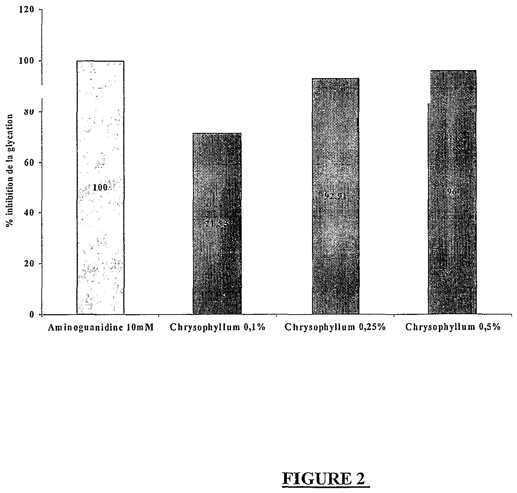

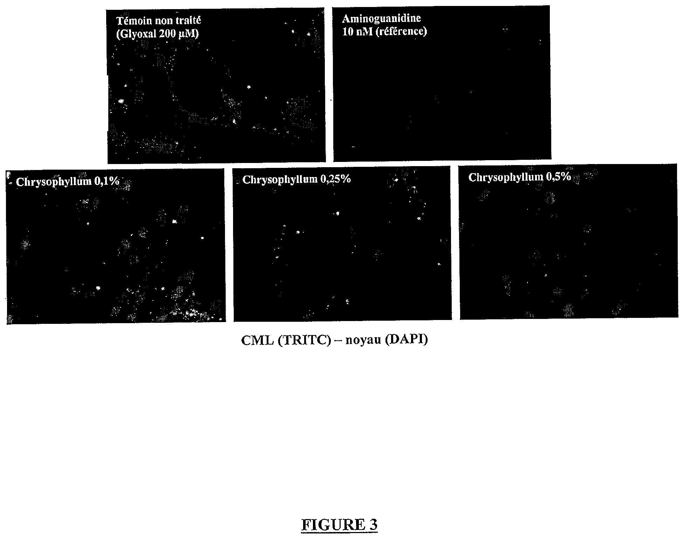

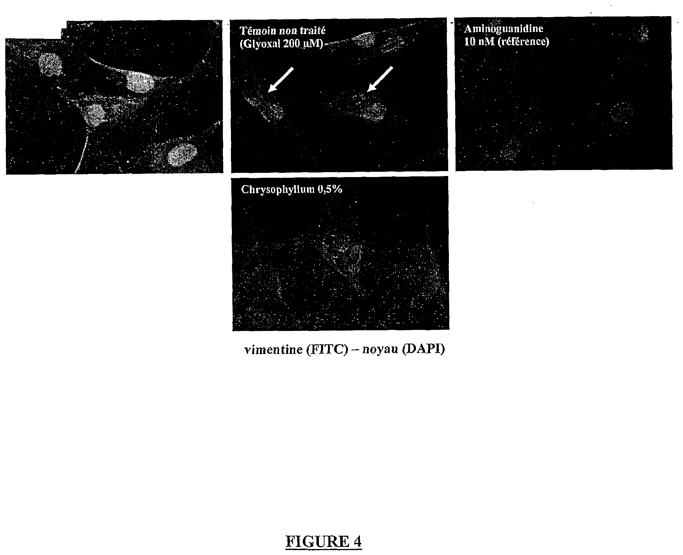

Les lamelles sont montées sur lame de verre puis observées au microscope à fluorescence (Nikon Eclipse 50i). Plusieurs champs de la population cellulaire sont pris en photo. Les clichés photographiques sont analysés par le logiciel d'analyse d'image Lucia. Les images de fluorescence FITC indiquent les régions de localisation de la vimentine. Les images de fluorescence TRITC indiquent les régions de localisation de CML. Les images de fluorescence DAPI indiquent les noyaux cellulaires et donc le nombre de cellules par champ. (voir figures 1 à 4)

### B.RESULTATS

### 1) Formation des CML

L'intensité de marquage de CML a été observée sur 10 clichés photographiques pour les conditions 'témoin non traité' et extraits de Chrysophyllum cainito aux 3 concentrations.

Cette intensité de fluorescence est rapportée au nombre de cellules par cliché.

Pour la condition 'traitement à l'aminoguanidine', référence positive, seulement 4 clichés ont été suffisants pour valider l'expérimentation. L'aminoguanidine, ayant inhibé totalement la génération des CML, induit l'absence de toute intensité de marquage.

Par l'analyse de la variance (Anova, ANalysis Of VAriance), on étudie les différences de moyenne entre populations. Cette méthode utilise des mesures de variance afin de déterminer le caractère significatif, ou non, des différences de moyenne mesurées sur les populations.

L'aminoguanidine, référence positive, inhibe totalement la formation des adduits carboxymétyliques de la lysine et valide notre expérimentation.

Dans ces conditions expérimentales, l'extrait de Chrysophyllum cainito inhibe de façon dose dépendante la formation des CML. Pour une dose optimale de 0,5% utilisée *in vitro*, l'extrait de Chrysophyllum cainito inhibe quasi totalement la formation des adduits engendrant la glycation.

### 2) Formation des CML

Dans ces conditions expérimentales, le traitement des fibroblastes humains dermiques par le glyoxal modifie la vimentine en ayant pour conséquence la redistribution du filament intermédiaire en agrégat périnucléaire (voir flèches).

L'extrait de Chrysophyllum cainito utilisé à la concentration 0,5% inhibe l'effet du glyoxal sur la vimentine.

### C.CONCLUSION

L'extrait de Chrysophyllum cainito a un effet protecteur de la vimentine vis-à-vis des composés dicarbonyl comme le glyoxal qui induit des modifications structurales de ce filament intermédiaire en y formant des adduits Carboxy Méthyliques de la Lysine ou CML.

L'inhibition de la glycation de la vimentine retarde le processus de vieillissement cellulaire et permet aux fibroblastes dermiques *in vitro* de conserver leur motilité et leur pouvoir contractile importants dans le processus de cicatrisation.

### II.EXEMPLES DE FORMULATION

Les pourcentages sont des pourcentages en poids par rapport au poids total de la composition.

**EMULSION E/H**

| | % |
|---|---|
| PEG 30 DIPOLYHYDROXYSTEARATE | 4,00 |
| TRIGLYCERIDES C16-C18 HYDROGENES | 5,00 |
| PEG-45 / DODECYL GLYCOL COPOLYMER | 1,20 |
| TRYGLYCERIDE C₈C₁₀ | 19,00 |
| HUILE DE VASELINE FLUIDE | 8,00 |
| GLYCOLS | 10,00 |
| EXTRAIT DE CHRYSOPHYLLUM | 2,50 |
| POLYOSES D'AVOINE | 1,00 |
| EAU DEMINERALISEE | Q.S.P 100 |

**LOTION**

| | % |
|---|---|
| GLYCOL | 2,00 |
| CHLORURE DE SODIUM | 1,00 |
| ETHANOL | 5,00 |
| EXTRAIT DE CHRYSOPHYLLUM | 0,50 |
| TROMETHAMINE | 0,80 |
| CONSERVATEURS | 0,50 |
| SOLUBILISANT | 0,30 |
| PARFUM | 0,10 |
| EAU DEMINERALISEE | Q.S.P 100 |

**GEL**

| | % |
|---|---|
| CARBOXYMETHYLCELLULOSE | 0,03 |
| CARBOMER | 0,60 |
| SOUDE | 0,17 |
| GLYCERINE | 5,00 |
| EXTRAIT DE CHRYSOPHYLLUM | 0,50 |
| D-PANTHENOL 75L | 0,30 |
| POLYOSES D'AVOINE | 1,00 |
| PALMITATE DE VITAMINE A | 0,10 |
| ACETATE DE TOCOPHEROL | 0,05 |
| HUILE DE RICIN HYDROGENEE PEG-40 | 1,35 |
| CHELATANT | 0,03 |
| CONSERVATEURS | 0,50 |
| COLORANT | 0,017 |
| PARFUM | 0,30 |
| EAU DEMINERALISEE | Q.S.P 100 |

**EMULSION H/E**

| | % |
|---|---|
| ACRYLOYL DIMETHYL TAURATE/VP COPOLYMER | 0,70 |
| HELIOGEL | 0,50 |
| GLYCOLS | 5,00 |
| CARBOMER | 0,50 |
| SOUDE | 0,055 |
| CARBOXYMETHYLCELLULOSE | 0,30 |
| TRYGLYCERIDE C₈C₁₀ | 4,50 |
| DICAPRYLYL CARBONATE | 6,00 |
| ESTER SYNTHETIQUE | 3, 00 |
| HUILE DE SILICONE | 1,50 |
| ACETATE DE TOCOPHEROL | 0,05 |
| PALMITATE DE VITAMINE A | 0,10 |
| D-PANTHENOL 75L | 0,20 |
| EXTRAIT DE CHRYSOPHYLLUM | 0,50 |
| CONSERVATEUR | 0,10 |
| CHELATANT | 0,20 |
| PARFUM | 0,30 |
| EAU DEMINERALISEE | Q.S.P 100 |

## Revendications

1. Utilisation d'une composition cosmétique comprenant au moins 0,1% d'extrait aqueux de la graine, de la pulpe et du péricarpe de fruit de Chrysophyllum cainito en poids de matière sèche par rapport au poids total de la composition, comme agent anti-glycation de la vimentine dans les fibroblastes dermiques humains pour prévenir et/ou retarder et/ou lutter contre le relâchement cutané et la perte d'élasticité des fibres cutanées.

2. Utilisation d'une composition cosmétique selon la revendication 1 pour prévenir et/ou retarder et/ou corriger la ptose des seins.

3. Utilisation selon la revendication 1 ou la revendication 2 dans laquelle ledit extrait aqueux est obtenu selon un procédé comprenant au moins les étapes suivantes :
- broyage des fruits secs en coupe
- ajout d'eau (température ambiante) afin de couvrir les fruits
- macération pendant au moins 24 h
- Filtration primaire.

4. Utilisation selon l'une quelconque des revendications précédentes dans laquelle ladite composition comprend de 0,1 à 10 % d'extrait aqueux de la graine, de la pulpe et du péricarpe de Chrysophyllum cainito en poids de matière sèche par rapport au poids total de la composition, de préférence de 0,1 à 5 % en poids d'extrait de Chrysophyllum cainito en poids de matière sèche par rapport au poids total de la composition.

5. Utilisation selon l'une quelconque des revendications précédentes dans laquelle ladite composition comprend en outre un ou plusieurs agents de formulation ou additifs tels que des adoucissants, des colorants, des agents filmogènes, des tensio-actifs, des parfums, des conservateurs, des émulsionnants, des huiles, des glycols, filtres UV, et des vitamines.

6. Utilisation selon l'une quelconque des revendications précédentes dans laquelle ladite composition se présente sous la forme d'un gel, d'une crème, d'une lotion, d'une huile, d'un lait ou d'un spray.

## Patentansprüche

1. Verwendung einer kosmetischen Zusammensetzung, umfassend mindestens 0,1 Gew.-% der Trockenmasse, bezogen auf das Gesamtgewicht der Zusammensetzung, wässrigen Extrakt des Samenkorns, des Fruchtfleisches und des Perikarps der Frucht von Chrysophyllum cainito als Antiglykationsmittel des Vimentins in den menschlichen dermalen Fibroblasten, um das Hauterschlaffen und den Elastizitätsverlust der Hautfasern zu verhindern und/oder zu verzögern und/oder dagegen anzukämpfen.

2. Verwendung einer kosmetischen Zusammensetzung gemäß Anspruch 1, um die Ptosis der Brüste zu verhindern und/oder zu verzögern und/oder zu korrigieren.

3. Verwendung gemäß Anspruch 1 oder Anspruch 2, wobei der wässrige Extrakt erhalten wird gemäß einem Verfahren, umfassend mindestens die folgenden Schritte:
- Zerkleinern geschnittener Trockenfrüchte
- Hinzufügen von Wasser (Raumtemperatur) um die Früchte zu bedecken
- Mazeration während mindestens 24 Stdn.
- Primärfiltration.

4. Verwendung gemäß einem der vorhergehenden Ansprüche, wobei die Zusammensetzung von 0,1 bis 10 Gew.-% der Trockenmasse, bezogen auf das Gesamtgewicht der Zusammensetzung, wässrigen Extrakt des Samenkorns, des Fruchtfleisches und des Perikarps von Chrysophyllum cainito umfasst, vorzugsweise von 0,1 bis 5 Gew.-% der Trockenmasse, bezogen auf das Gesamtgewicht der Zusammensetzung, Extrakt von Chrysophyllum cainito.

5. Verwendung gemäß einem der vorhergehenden Ansprüche, wobei die Zusammensetzung des Weiteren ein oder mehrere Formulierungsmittel oder Zusatzstoffe, wie Weichmacher, Farbstoffe, Filmbildner, Tenside, Duftstoffe, Konservierungsmittel, Emulgatoren, Öle, Glycole, UV-Filter und Vitamine, umfasst.

6. Verwendung gemäß einem der vorhergehenden Ansprüche, wobei die Zusammensetzung in der Form eines Gels, einer Creme, einer Lotion, eines Öls, einer Milch oder eines Sprays vorliegt.

## Claims

1. Use of a cosmetic composition comprising at least 0.1% aqueous extract of the seed, the pulp and the pericarp of the fruit of *Chrysophyllum cainito,* by weight of dry matter with respect to the total weight of the composition, as an anti-glycation agent of vimentin in human skin fibroblasts, in order to prevent and/or delay and/or combat skin sagging and loss of elasticity of the cutaneous fibres.

2. Use of a cosmetic composition according to claim 1 for preventing and/or delaying and/or correcting breast ptosis.

3. Use according to claim 1 or claim 2, wherein said aqueous extract is obtained according to a method comprising at least the following steps:
- grinding of sliced dried fruit
- addition of water (at ambient temperature) in order to cover the fruits
- maceration for 24 hours
- primary filtration.

4. Use according to any one of the preceding claims, wherein said composition comprises 0.1 to 10% aqueous extract of the seed, the pulp and the pericarp of *Chrysophylum cainito* by weight of dry matter with respect to the total weight of the composition, preferably 0.1 to 5% of *Chrysophyllum cainito,* extract by weight of dry matter with respect to the total weight of the composition.

5. Use according to any one of the preceding claims, wherein said composition further comprises one or more formulation agents or additives such as softeners, dyes, film forming agents, surfactants, perfumes, preservatives, emulsifiers, oils, glycols, UV filters and vitamins.

6. Use according to any one of the preceding claims, wherein said composition is in the form of a gel, cream, lotion, oil, milk or spray.
